# EUROPEAN PATENT SPECIFICATION

(11) **EP 3 007 743 B1**
(45) Date of publication and mention of the grant of the patent: **11.12.2019**
(21) Application number: 14732445.3
(22) Date of filing: 06.06.2014
(51) Int. Cl.: A61M 5/00, A61M 5/19, A61M 5/315, A61M 39/10, A61M 39/12, A61B 17/00, A61M 39/00

(54) **SYRINGE PAIR ASSEMBLY FOR BIOLOGICAL FLUIDS**
SPRITZENPAARANORDNUNG FÜR BIOLOGISCHE FLÜSSIGKEITEN
ENSEMBLE SERINGUES APPAIRÉES POUR LIQUIDES BIOLOGIQUES

(30) Priority: 10.06.2013 WO PCT/NL2013/050410
(43) Date of publication of application: 20.04.2016
(73) Proprietor: Recuperate Medical B.V., 9301 NZ Roden (NL)
(72) Inventor: KUPER, Hendericus Johannes Maria, 9301 NZ Roden (NL)
(74) Representative: V.O.
(86) International application number: PCT/NL2014/050365
(87) International publication number: WO 2014/200338

(56) References cited:
- EP-A2- 2 005 934
- WO-A1-01/41666
- WO-A1-2011/109915
- WO-A1-2013/063476
- US-A1- 2006 253 082
- US-A1- 2011 319 930
- US-A1- 2013 041 258
- US-B1- 6 679 300

## Description

The invention relates to a syringe pair assembly for biological fluids comprising two syringes, each syringe comprising a barrel, a nozzle and a plunger, a barrel holding frame for holding the barrels, a plunger holder for holding the plungers, a fitting with tubing, which fitting is adapted for frictionally holding the nozzles of the two syringes and for linking the two nozzles to the tubing, a retaining member securing the barrel-holding frame to the fitting, a membrane enclosing the two syringes and having one end sealed over the fitting with tubing and another closed end opposite the one end deployed about the syringe plungers and adapted for allowing an operator to grasp and extend the plunger ends during filling of the syringes without exposure to ambient conditions, said membrane being formed from parts which are separably sealed together.

Such a syringe pair assembly is disclosed in US-2004/0140012. This known syringe pair assembly comprises a spring-biased plastic retaining member securing the barrel-holding frame to the fitting. The membrane is deployed about the syringe plungers to allow an operator to grasp and extend the plunger end during filling of the two syringes without exposure to ambient conditions. The free or plunger end of the membrane is formed from two parts which are sealed together but are separable by peeling apart to expose a plunger connector. To remove the two syringes, the spring-biased plastic retaining member and the barrel-holding frame from the fitting an operator has to depress the spring-biased plastic retaining member toward the syringes to lift the retaining member from a mating catch on the fitting. Then the syringes have to be twisted and to be pulled away from the fitting allowing the two syringes to reside loose within the membrane. The free end of the membrane is then peeled apart and the two syringes can be removed to allow use in therapeutic procedure. However, it appears that the removal of the two syringes from the fitting by of the two step disconnection sometimes results in that the two syringes are actually not disconnected from the fitting. It then can happen that a physician or any person per physician's instruction who wants to use the syringe pair realizes that there is no actual disconnection and has to perform additional handling which not only costs time but also leads to annoyance. This can be detrimental for the result of the therapeutic procedure. In addition, after the two syringes have been correctly disconnected from the fitting it can happen that by inappropriately or unintentionally handling the two syringe plungers content of the syringes can escape out off the barrel before the intended time of use. This leads to content being present within the membrane and sometimes also on the plunger ends or barrels which can negatively influence the handling of the syringe pair, can lead to an insufficient amount of content for the therapeutic procedure or can result therein that sterility is compromised. Each of these situations is not desired during therapeutic procedure. Furthermore, it can happen that during peeling off the membrane the syringe pair is dropped on the floor, in particular when the disconnection is not effected properly and additional twisting is necessary to realize a proper disconnection making the syringe pair unsuitable for use. In this case, which is not a theoretical situation but appears to happen frequently in the hectic of therapeutic procedure, a new syringe pair assembly has to be ordered and often first has to be thawed which not only increases costs but leads to valuable time being lost for completing the therapeutic procedure.

Further features of syringe assemblies can be found in US 2006/253082, WO 2013/063476, US 2011/319930, US 2013/041258, EP 2 005 934, WO 2011/109915 and WO 01/41666.

Accordingly, it is an object of the present invention to provide a syringe pair assembly which can be handled more easily and efficiently in a surgical setting and which maintains sterility better.

The above object is achieved in accordance with the invention by providing a syringe pair assembly for biological fluids comprising two syringes, each syringe comprising a barrel, a nozzle and a plunger, a barrel holding frame for holding the barrels, a plunger holder for holding the plungers, a fitting with tubing, which fitting is adapted for frictionally holding the nozzles of the two syringes and for linking the two nozzles to the tubing, a retaining member securing the barrel-holding frame to the fitting, a membrane enclosing the two syringes and having one end sealed over the tubing and another closed end opposite the one end deployed about the syringe plungers and adapted for allowing an operator to grasp and extend the plunger ends during filling of the syringes without exposure to ambient conditions, said membrane being formed from parts which are separably sealed together, which is characterized in that the fitting is formed by a first fitting part and a second fitting part which are releasably connected to each other by means of a connection member, the tubing and the one end of the membrane being attached to the first fitting part, the second fitting part being adapted for frictionally holding the nozzles of the two syringes and in that the assembly further comprises an inner membrane positioned within the membrane, said inner membrane enclosing the two syringes and having one end sealed to the second fitting part and another closed end opposite the one end deployed about the syringe plungers and adapted for allowing an operator to grasp and extend the plunger ends during filling of the syringes without exposure to ambient conditions, said inner membrane being formed from parts which are separably sealed together. By assembling the fitting out of two fitting parts disconnection of the first from the second part can be detected more clearly while the nozzles are still frictionally held by the second part after the first and the second part have been disconnected. In this manner the two syringes with the second part can be displaced to the operation table in a user friendly manner which guarantees a better sterility. Although just before use of the syringe pair an additional handling is necessary to disconnect the two syringes from the second part, this in practice appears to increase operating efficiency. Further, by in accordance with the invention using an inner membrane the membrane (which then is an outer membrane) can be peeled open to remove the second fitting part with the two syringes attached thereto, while the two syringes are still enclosed by the inner membrane. Thus even if the inner membrane with the two syringes drops down during opening of the membrane sterility is not compromised. The inner membrane and the two syringes can thus be handled in a sterile way and e.g. be placed on the operation table without exposing the syringes to ambient conditions. The inner membrane can be formed by an enclosure which can be opened easily with a minimum of force required.

From US-A-2006/0253082 a dispensing assembly with a syringe pair assembly for dispensing two separate fluids to a treatment site is known per se, in which dispensing assembly a fitting is composed of two interconnectable parts. This known dispensing assembly does not comprise a membrane or bag covering the syringe pair assembly.

In a preferred embodiment of the assembly according to the invention the retaining member releasably secures the barrel-holding frame to the second fitting part. For actually using the syringe pair in a surgical setting the inner membrane can be opened and thereafter or prior to opening of the inner membrane the retaining member can be operated upon to disconnect the nozzles from the second fitting part. The retaining member can be formed as any known easy to operate retaining member, such as a snap connection or latch and catch mechanism so that removing the two syringes from the second fitting part can be effected safely.

In an embodiment of a syringe pair assembly according to the invention the connection member is adapted for connecting the first and the second fitting part under pretension. In this manner an operator does not need to apply much force to disconnect the first and second fitting parts which increases ease of handling. In addition the pretension present during connection between the first and second fitting part can ensure a reproducible separation of the two fitting parts since the pretension pushes the two fitting parts apart during disconnection. The construction of the fitting can be relatively simple when said connection member is adapted for connecting the first and the second fitting part under a pretension exerting a force in one direction only. In addition, in this manner disconnection of the two fitting parts can be detected even more clearly.

In a particular advantageous embodiment of a syringe pair assembly according to the invention the connection member comprises a first connection member part provided on the first fitting part and a second connection member part provided on the second fitting part, one of the first and second connection member parts being formed by a pivotal latch and the other one of the first and second connection member parts being formed by a catch, said pivotal latch and said catch being adapted for connecting the first and the second fitting part under pretension, and said pivotal latch comprising a push lever. In this manner an operator only has to press the push lever to effect a reproducible disconnection of the two fitting parts. Such a simple handling can be beneficial and user friendly during therapeutic procedure.

To even increase the certainty that the two fitting parts are disconnected for each syringe the first fitting part comprises a protruding pipe at a side of the first fitting part opposite the tubing and the second fitting part comprises a hole receiving the protruding pipe, said hole being in fluid communication with the nozzle of the respective syringe. In this manner disconnection of the two fitting parts can provide a visual check for the operator the ensure that disconnection has taken place since the pipes, optionally provided with a bright color, are visible after disconnection. In addition the pipes and the holes ensure that disconnection of the two fitting parts can only take place in a single direction, which increases safety of use.

In a particular advantageous embodiment of a syringe pair assembly according to the invention the second fitting part is provided with a valve for each nozzle, said valve being adapted for allowing fluid communication between the tubing and the respective nozzle when the first fitting part and a second fitting part are connected to each other and for shutting off the nozzle when the first fitting part and the second fitting part are disconnected from each other. By assembling the fitting out of two fitting parts it is possible to provide the second fitting part with such a valve, which can prevent inadvertent spillage of content from the syringes. This provides a saving in costs and furthermore increases the sterility of the assembly during use. Such a shutting off of the valve can be realized in a constructional simple but reliable manner when in each hole of the second fitting part a valve is positioned and when the connection member, the first and second fitting part and the valve are adapted to automatically shut off the hole when the first fitting part and the second fitting part which are disconnected from each other. The valve can be a flexible valve sleeve receiving and surrounding the protruding pipe.

Preferably each valve is a rotatable valve mounted under pretension in the respective hole of the second fitting part. In this manner it is possible to construct the syringe pair assembly such that when the protruding pipe is inserted in the respective hole, the valve mounted in the hole is pushed inwards and simultaneously rotates to a position in which fluid can be passed into the barrel, while as a result of the pretension when the protruding pipe is retracted from the hole the valve is rotated to a forward and closing position in which the hole is shut off.

In an embodiment of a syringe pair assembly according to the invention which can be constructed in a particularly simple manner while providing a safe, sterile and reliable handling the first fitting part has a first flat surface, in that the second fitting part has a second flat surface, the first and second flat surface facing each other and being parallel to each other when the first and second fitting part are connected to each other, wherein the connection member is adapted for connecting the first and the second fitting part under pretension which pretension is directed at least substantially transverse to the first and second flat surfaces.

The invention also relates to a syringe assembly wherein instead of two syringes the syringe assembly comprises one, three or more syringes.

To further clarify various aspects of embodiments of the present disclosure and additional features and advantages of the embodiments, a more particular description of various aspects and features will be rendered by reference to specific embodiments thereof which are illustrated in the appended drawings. It is appreciated that these drawings depict only typical embodiments of the disclosure and are therefore not to be considered limiting its scope, nor are the figures necessarily drawn to scale.

The embodiments herein will be described and explained with additional specificity and detail through the use of the accompanying drawings in which as biological fluids components derived from human plasma for fibrinogen based sealant are used to elucidate the invention:
Fig. 1A schematically shows a biological fluid bag loading system comprising a loading station and a number of syringe pair assemblies according to the invention attached to the loading station in a perspective view;
Fig. 1B schematically shows the biological fluid bag loading system of Figure 1A in front view;
Fig. 1C schematically shows the biological fluid bag loading system of Figure 1A in side view;
Fig. 2A schematically shows a front view of a syringe pair assembly according to the invention connected to a dispensing manifold;
Fig. 2B schematically shows the syringe pair assembly of Fig. 2A in side view;
Fig. 2C schematically shows the syringe pair assembly of Fig. 2B in cross section;
Fig. 2D schematically shows an embodiment of a syringe pair assembly in which the valve is formed by a valve sleeve, in which the part shown in Fig. 2D corresponds to part IID of Fig. 2C in an enlarged scale in the situation in which the first and second fitting parts are connected to each other;
Fig. 2E schematically shows part IIE of Fig. 2D in an enlarged scale;
Fig. 3A schematically shows a front view of another embodiment of a syringe pair assembly according to the invention in which the valve is formed by a rotary valve mounted under pretension in which the rotary valve is in a position allowing flow of fluid into the barrels;
Fig. 3B schematically shows part IIIB of Fig. 3A in an enlarged scale;
Fig. 4A schematically shows the syringe pair assembly of Fig. 3A in a situation in which the first and second fitting parts are disconnected from ach other and the rotary valve is in a shutting off position; and
Fig. 4B schematically shows part IVB of Fig. 4A in an enlarged scale.

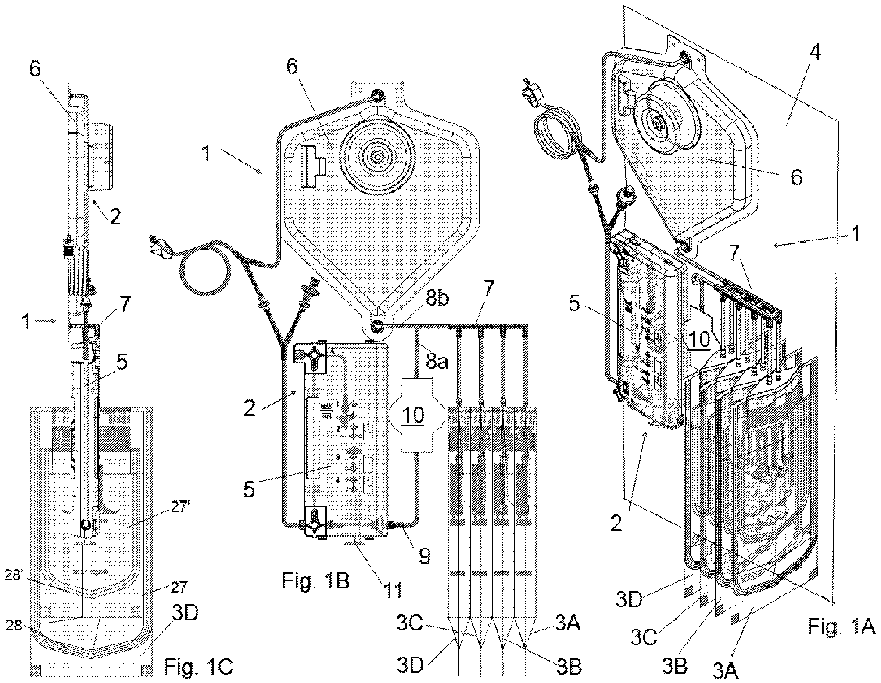

Considering the drawings, wherein like reference numerals denote like parts throughout the various drawing figures, reference numeral 1 as shown in FIG. 1 is directed to a loading system for a biological fluid bag, which system comprises a loading station 2 for a biological fluid bag and a number of syringe pair assemblies 3A, 3B, 3C, 3D according to the invention.

In its essence, the loading station 2 includes a support 4, to which the following are mounted: a thrombin processing unit 5, a clotting and fluid proteins processing unit 6 and a dispensing manifold 7. Each unit 5, 6 has a separate dispensing line 8a, 8b to the dispensing manifold 7 as shown in FIG. 1B, to maintain sequestration of each component of the biological sealant. An outlet 9 connected to the thrombin processing unit 5 leads into a reserve vessel 10, whereby pressure from a thrombin syringe 11 causes thrombin to enter the reserve vessel 10. The dispensing manifold 7 is preferably oriented to load a plurality of syringe pair assemblies 3, in the shown embodiment four although other numbers of syringe pair assemblies 3 are also applicable, with components of the biological sealant. As is clearly shown in Figure 1C an inner membrane 27' having a closed end 28' and an outer membrane 27 having a closed end 28 enclose the two syringes 12; 13 of a syringe pair assembly 3D. The membranes 27, 27' are formed from parts which are separably sealed together, e.g. by peelable seals.

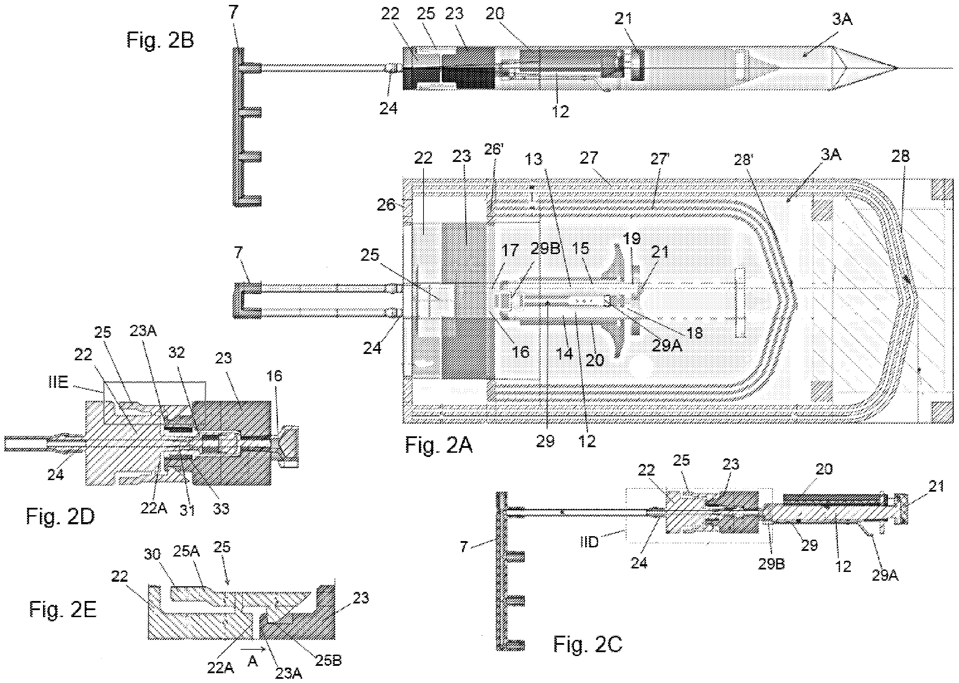

An embodiment of one of the syringe pair assemblies, in this case 3A, according to the invention will be described in more detail with regard to Figures 2A-E.

The syringe pair assembly 3A for biological fluids, in the shown exemplary embodiment biological sealant components, comprises two syringes 12; 13. Each syringe 12; 13 comprises a barrel 14; 15, a nozzle 16; 17 and a plunger 18; 19. A barrel holding frame 20 is provided for holding the barrels 14; 15 and a plunger holder 21 holds the plungers 18; 19.

The syringe pair assembly 3A further comprises a fitting 22, 23 with tubing 24, which fitting is formed by a first fitting part 22 and a second fitting part 23 which are releasably connected to each other by means of a connection member 25. The tubing 24 and one end 26 of the outer membrane 27 are attached to the first fitting part 22. The outer membrane 27 encloses the two syringes 12; 13 and has the one end 26 sealed over the tubing 24 and another closed end 28 opposite the one end 26 deployed about the syringe plungers 18; 19 and adapted for allowing an operator to grasp and extend the plunger ends (indicated with broken lines) during filling of the syringes 12; 13 without exposure to ambient conditions. The outer membrane 27 is formed from parts which are separably sealed together, e.g. by peelable seals. The inner membrane 27', positioned within the outer membrane 27, also encloses the two syringes 12; 13 and has one end 26' sealed to the second fitting part 23 and another closed end 28' opposite the one end 26' deployed about the syringe plungers 18; 19 and adapted for allowing an operator to grasp and extend the plunger ends (indicated with broken lines) during filling of the syringes 12; 13 without exposure to ambient conditions.

The second fitting part 23 is adapted for frictionally holding the nozzles 16; 17 of the two syringes 12; 13. The fitting 22, 23 is further adapted for linking the two nozzles 16; 17 to the tubing 24 which tubing 24, during filling, is in fluid communication with the dispensing manifold 7. A retaining member 29 secures the barrel-holding frame 20 to the fitting 22, 23.

The connection member 25 is adapted for connecting the first fitting part 22 and the second fitting part 23 under pretension as is indicated in Figs. 2D and 2E. In this manner an operator does not need to apply much force to disconnect the first and second fitting parts 22, 23 which increases ease of handling. In addition the pretension present during connection between the first and second fitting parts 22, 23 ensures a reproducible separation of the two fitting parts 22, 23 since the pretension pushes the two fitting parts 22, 23 apart during disconnection. In the embodiment shown in Figs. 2D and 2E the construction of the fitting parts and the connection member is such that the pretension exerts a force in one direction, indicated by arrow A only. As can be seen in these Figures the first fitting part 22 has a first flat surface 22A and the second fitting part 23 has a second flat surface 23A, which flat surfaces 22A, 23A face each other and are parallel to each other when the first and second fitting parts 22, 23 are connected to each other. Consequently, the direction of the pretension points at least substantially transverse to the first and second flat surfaces 22A, 23A.

In the embodiment shown in Figs. 2 the connection member 25 comprises a first connection member part 25A provided on the first fitting part 22 and a second connection member part 25B provided on the second fitting part 23 (see Fig. 2E). The first connection member part 25A is formed by a pivotal latch and the other second connection member part 25B is formed by a catch, which pivotal latch 25A and catch 25B are adapted for connecting the first and the second fitting parts 22, 23 under pretension. The pivotal latch 25A comprising a push lever 30 which is easily accessible by an operator who only has to press the push lever 30 to effect a reproducible disconnection of the two fitting parts 22, 23.

The first fitting part 22 comprises a protruding pipe 31 (Fig. 2D) at a side of the first fitting part opposite the tubing 24 and the second fitting part 23 comprises a hole 32 receiving the protruding pipe 31. The hole 32 is in fluid communication with the nozzle 16 of the syringe 12. In this manner disconnection of the two fitting parts 22, 23 can provide a visual check for the operator the ensure that disconnection has taken place since the pipe 31, optionally provided with a bright (red) color, is visible after disconnection. Please note that the same applies for the other syringe 13.

Also visible in Fig. 2D is a valve 33 which is provided in the second fitting part 23. The valve 33 is in this embodiment formed by a flexible valve sleeve receiving and surrounding the protruding pipe 31. The valve sleeve 33 is adapted for allowing fluid communication between the tubing 24 and the nozzle 16 when the first fitting part 22 and a second fitting part 23 are connected to each other. The valve sleeve 33 automatically shuts off the nozzle 16 (i.e. interrupts the fluid communication) when the first fitting part 22 and the second fitting part 23 are disconnected from each other and thus prevents inadvertent spillage of content from the syringe 12.

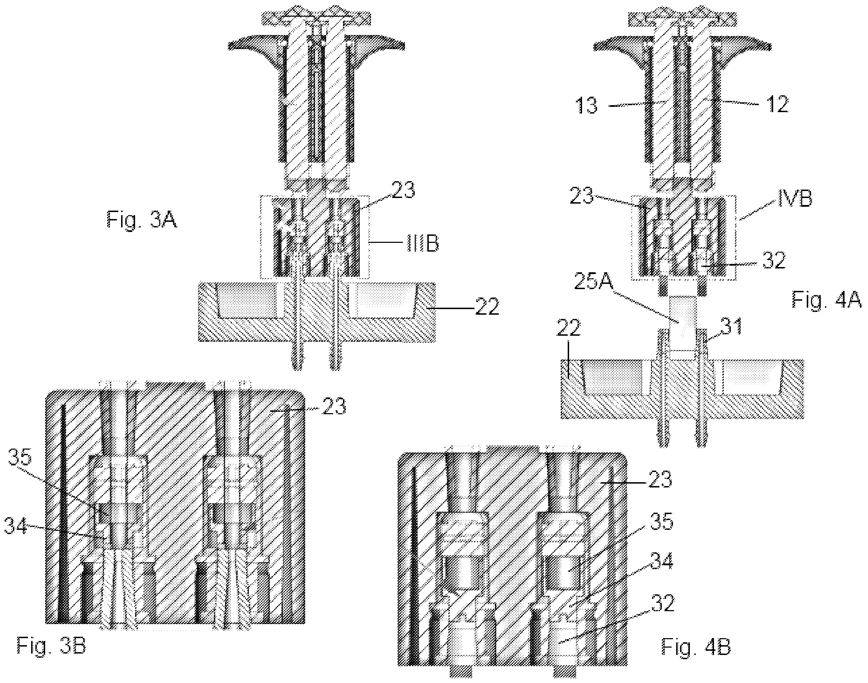

In Fig. 3A another embodiment of a syringe pair assembly according to the invention is shown, in which drawing the membranes are left out for convenience. In this embodiment the valve is formed by a rotary valve 34 which is mounted under pretension in the hole 32 of the second fitting part 23. In the shown embodiment the pretension is realized by an elastic member 35 . In the situation shown in Figures 3A and 3B the protruding pipe 31 pushes the rotary valve 34 into the hole 32 and thereby rotates the rotary valve 34 in an open position such that the barrels can be filled. In Figures 4A and 4B the situation is shown in which the first fitting part 22 and the second fitting part 23 are disconnected from each other. As a result of the pretension provided by member 35 the rotary valve is pushed forward into a position (clearly visible in Figure 4B) in which the rotary valve 34 shuts off the hole 32, thereby preventing spillage since the rotary valve 34 automatically shuts off the nozzle 16 (i.e. interrupts the fluid communication) when the first fitting part 22 and the second fitting part 23 are disconnected from each other and thus prevents inadvertent spillage of content from the syringe 12. Please note that the same applies for the syringe 13.

The retaining member 29 releasably secures the barrel-holding frame 20 to the second fitting part 23 and can be operated upon, e.g. by pushing the protruding handle portion 29A (Figs. 2A and 2C) to disconnect the nozzles 16, 17 from the second fitting part 23. In this embodiment the retaining member 29 comprises a latch and catch mechanism 29B so that removing the two syringes from the second fitting part can be effected easily and safely.

## Claims

1. Syringe pair assembly (3; 3A, 3B, 3C, 3D) for biological fluids comprising two syringes (12, 13), each syringe (12, 13) comprising a barrel (14, 15), a nozzle (16, 17) and a plunger (18, 19), a barrel holding frame (20) for holding the barrels (14, 15), a plunger holder (21) for holding the plungers (18, 19), a fitting (22, 23) with tubing (24), which fitting (22, 23) is adapted for frictionally holding the nozzles (16, 17) of the two syringes (12, 13) and for linking the two nozzles (16, 17) to the tubing (24), a retaining member (29) securing the barrel-holding frame to the fitting (22, 23), a membrane (27) enclosing the two syringes (12, 13) and having one end (26) sealed over the fitting (22, 23) with tubing (24) and another closed end (28) opposite the one end (26) deployed about the syringe plungers (18, 19) and adapted for allowing an operator to grasp and extend the plunger ends during filling of the syringes (12, 13) without exposure to ambient conditions, said membrane being formed from parts (27, 27') which are separably sealed together, **characterized in that** the fitting (22, 23) is formed by a first fitting part (22) and a second fitting part (23) which are releasably connected to each other by means of a connection member (25), the tubing (24) and the one end (26) of the membrane (27) being attached to the first fitting part (22), the second fitting part (23) being adapted for frictionally holding the nozzles (16, 17) of the two syringes (12, 13), and **in that** the assembly (3; 3A, 3B, 3C, 3D) further comprises an inner membrane (27') positioned within the membrane (27), said inner membrane (27') enclosing the two syringes (12, 13) and having one end (26') sealed to the second fitting part (23) and another closed end (28') opposite the one end (26') deployed about the syringe plungers (18, 19) and adapted for allowing an operator to grasp and extend the plunger ends during filling of the syringes (12, 13) without exposure to ambient conditions, said inner membrane (27') being formed from parts which are separably sealed together.

2. Syringe pair assembly (3; 3A, 3B, 3C, 3D) according to claim 1, **characterized in that** the connection member (25) is adapted for connecting the first and the second fitting part (22, 23) under pretension.

3. Syringe pair assembly (3; 3A, 3B, 3C, 3D) according to claim 1 or 2, **characterized in that** said connection member (25) is adapted for connecting the first and the second fitting part (22, 23) under a pretension exerting a force in one direction only

4. Syringe pair assembly (3; 3A, 3B, 3C, 3D) according to claim 3, **characterized in that** the connection member (25) comprises a first connection member part (25A) provided on the first fitting part (22) and a second connection member part (25B) provided on the second fitting part (23), one (25A) of the first and second connection member parts (25A, 25B) being formed by a pivotal latch (25A) and the other one (25B) of the first and second connection member parts (25A, 25B) being formed by a catch (25B), said pivotal latch (25A) and said catch (25B) being adapted for connecting the first and the second fitting part (22, 23) under pretension, and said pivotal latch (25A) comprising a push lever (30).

5. Syringe pair assembly (3; 3A, 3B, 3C, 3D) according to any one of the preceding claims, **characterized in that** for each syringe (12, 13) the first fitting part (22) comprises a protruding pipe (31) at a side of the first fitting part (22) opposite the tubing (24) and the second fitting part (23) comprises a hole (32) receiving the protruding pipe (31), said hole (32) being in fluid communication with the nozzle (16, 17) of the respective syringe (12, 13).

6. Syringe pair assembly (3; 3A, 3B, 3C, 3D) according to any one of the preceding claims, **characterized in that** the second fitting part (23) is provided with a valve (33, 34) for each nozzle (16, 17), said valve (33, 34) being adapted for allowing fluid communication between the tubing (24) and the respective nozzle (16, 17) when the first fitting part (22) and the second fitting part (23) are connected to each other and for shutting off the nozzle (16, 17) when the first fitting part (22) and the second fitting part (23) are disconnected from each other.

7. Syringe pair assembly (3; 3A, 3B, 3C, 3D) according to claim 5 and 6, **characterized in that** in each hole (32) of the second fitting part (23) a valve (33, 34) is positioned, and **in that** the connection member (25), the first and second fitting part (22, 23) and the valve (33, 34) are adapted to automatically shut off the hole (32) when the first fitting part (22) and the second fitting part (23) are disconnected from each other.

8. Syringe pair assembly (3; 3A, 3B, 3C, 3D) according to claim 6 or 7, **characterized in that** the valve (33, 34) is a flexible valve sleeve (33) receiving and surrounding the protruding pipe (31).

9. Syringe pair assembly (3; 3A, 3B, 3C, 3D) according to claim 6 or 7, **characterized in that** each valve (33, 34) is a rotatable valve (34) mounted under pretension in the respective hole (32) of the second fitting part (23).

10. Syringe pair assembly (3; 3A, 3B, 3C, 3D) according to any one of the claims, **characterized in that** the first fitting part (22) has a first flat surface (22A), **in that** the second fitting part (23) has a second flat surface (23A), the first and second flat surface (22A, 23A) facing each other and being parallel to each other when the first and second fitting part (22, 23) are connected to each other, and **in that** the connection member (25) is adapted for connecting the first and the second fitting part (22, 23) under pretension which pretension is directed at least substantially transverse to the first and second flat surfaces (22A, 23A).

11. Syringe pair assembly (3; 3A, 3B, 3C, 3D) according to any one of the preceding claims, **characterized in that** the retaining member (29) releasably secures the barrel-holding frame to the second fitting part (23).

12. Syringe pair assembly (3; 3A, 3B, 3C, 3D) according to any one of the preceding claims, wherein instead of two syringes (12, 13) the syringe pair assembly (3; 3A, 3B, 3C, 3D) comprises one, three or more syringes.

## Patentansprüche

1. Spritzenpaaranordnung (3; 3A, 3B, 3C, 3D) für biologische Flüssigkeiten mit zwei Spritzen (12, 13), wobei jede Spritze (12, 13) einen Zylinder (14, 15), eine Düse (16, 17) und einen Kolben (18, 19), einen Zylinderhalterahmen (20) zum Halten der Zylinder (14, 15), einen Kolbenhalter (21) zum Halten der Kolben (18, 19), einen Anschluss (22, 23) mit Schlauch (24), welcher Anschluss (22, 23) zum reibschlüssigen Halten der Düsen (16, 17) der beiden Spritzen (12, 13) und zum Verbinden der beiden Düsen (16, 17) mit dem Schlauch (24) ausgebildet ist, ein Halteelement (29), das den Zylinderhalterahmen an dem Anschluss (22, 23) befestigt, eine Membran (27), die die beiden Spritzen (12, 13) umschließt und ein Ende (26), das über dem Anschluss (22, 23) mit einem Schlauch (24) versiegelt ist und ein anderes geschlossenes Ende (28) gegenüber dem einen Ende (26) aufweist, das um die Spritzenkolben (18, 19) entfaltet wird und so ausgelegt ist, dass es einem Bediener erlaubt, die Kolbenenden während des Befüllens der Spritzen (12, 13) zu greifen und auszufahren, ohne sie Umgebungsbedingungen auszusetzen, umfasst, wobei die Membran aus Teilen (27, 27') gebildet ist, die trennbar miteinander versiegelt sind, **dadurch gekennzeichnet, dass** der Anschlussteil (22, 23) durch einen ersten Anschlussteil (22) und einen zweiten Anschlussteil (23) gebildet wird, die mittels eines Verbindungselements (25) miteinander lösbar verbunden sind, wobei der Schlauch (24) und das eine Ende (26) der Membran (27) an dem ersten Anschlussteil (22) angebracht sind, wobei der zweite Anschlussteil (23) ausgelegt ist, um die Düsen (16, 17) der beiden Spritzen (12, 13) reibschlüssig zu halten, und **dadurch, dass** die Anordnung (3; 3A, 3B, 3C, 3D) ferner eine innere Membran (27') umfasst, die in der Membran (27) positioniert ist, wobei die innere Membran (27') die zwei Spritzen (12, 13) umschließt und ein Ende (26') aufweist, das an dem zweiten Anschlussteil (23) versiegelt ist und wobei ein anderes geschlossenes Ende (28') gegenüber dem einen Ende (26') um die Spritzenkolben (18, 19) entfaltet ist und so ausgelegt ist, dass es einem Bediener erlaubt, die Kolbenenden während des Befüllens der Spritzen (12, 13) zu greifen und aufzufahren, ohne sie den Umgebungsbedingungen auszusetzen, wobei die innere Membran (27') aus Teilen gebildet ist, die trennbar miteinander versiegelt sind.

2. Spritzenpaaranordnung (3; 3A, 3B, 3C, 3D) nach Anspruch 1, **dadurch gekennzeichnet, dass** das Verbindungselement (25) dazu ausgelegt ist, den ersten und den zweiten Anschlussteil (22, 23) unter Vorspannung zu verbinden.

3. Spritzenpaaranordnung (3; 3A, 3B, 3C, 3D) nach Anspruch 1 oder 2, **dadurch gekennzeichnet, dass** das Verbindungselement (25) zum Verbinden des ersten und des zweiten Anschlussteils (22, 23) unter einer Vorspannung ausgelegt ist, die eine Kraft nur in einer Richtung ausübt

4. Spritzenpaaranordnung (3; 3A, 3B, 3C, 3D) nach Anspruch 3, **dadurch gekennzeichnet, dass** das Verbindungselement (25) einen ersten Verbindungselementteil (25A) umfasst, der an dem ersten Anschlussteil (22) vorgesehen ist, und einen zweiten Verbindungselementteil (25B), der an dem zweiten Anschlussteil (23) vorgesehen ist, wobei einer (25A) des ersten und des zweiten Verbindungselementteile (25A, 25B) durch eine Schwenkverriegelung (25A) gebildet ist und der andere (25B) des ersten und des zweiten Verbindungselementteile (25A, 25B) durch eine Arretierung (25B) gebildet ist, wobei die Schwenkverriegelung (25A) und die Arretierung (25B) ausgelegt sind, um den ersten und den zweiten Anschlussteil (22, 23) unter Vorspannung zu verbinden und die Schwenkverriegelung (25A) einen Druckhebel (30) aufweist.

5. Spritzenpaaranordnung (3; 3A, 3B, 3C, 3D) nach einem der vorhergehenden Ansprüche, **dadurch gekennzeichnet, dass** für jede Spritze (12, 13) der erste Anschlussteil (22) ein vorstehendes Rohr (31) an einer Seite des ersten Anschlussteils (22) gegenüber dem Schlauch (24) umfasst und der zweite Anschlussteil (23) eine Bohrung (32) umfasst, die das vorstehende Rohr (31) aufnimmt, wobei die Bohrung (32) in Fluidverbindung mit der Düse (16, 17) der jeweiligen Spritze (12, 13) steht.

6. Spritzenpaaranordnung (3; 3A, 3B, 3C, 3D) nach einem der vorhergehenden Ansprüche, **dadurch gekennzeichnet, dass** das zweite Anschlussteil (23) mit einem Ventil (33, 34) für jede Düse (16, 17) versehen ist, wobei das Ventil (33, 34) ausgelegt ist, um eine Fluidverbindung zwischen dem Schlauch (24) und der jeweiligen Düse (16, 17) zu ermöglichen, wenn der erste Anschlussteil (22) und der zweite Anschlussteil (23) miteinander verbunden sind und um die Düse (16, 17) abzusperren, wenn der erste Anschlussteil (22) und der zweite Anschlussteil (23) voneinander getrennt sind.

7. Spritzenpaaranordnung (3; 3A, 3B, 3C, 3D) nach Anspruch 5 und 6, **dadurch gekennzeichnet, dass** in jeder Bohrung (32) des zweiten Anschlussteils (23) ein Ventil (33, 34) positioniert ist, und dass das Verbindungselement (25), der erste und der zweite Anschlussteil (22, 23) und das Ventil (33 , 34) ausgelegt sind, um die Bohrung (32) automatisch abzusperren, wenn der erste Anschlussteil (22) und der zweite Anschlussteil (23) voneinander getrennt werden.

8. Spritzenpaaranordnung (3; 3A, 3B, 3C, 3D) nach Anspruch 6 oder 7, **dadurch gekennzeichnet, dass** das Ventil (33, 34) eine flexible Ventilhülse (33) ist, die das vorstehende Rohr (31) aufnimmt und umgibt.

9. Spritzenpaaranordnung (3; 3A, 3B, 3C, 3D) nach Anspruch 6 oder 7, **dadurch gekennzeichnet, dass** jedes Ventil (33, 34) ein drehbares Ventil (34) ist, das in der jeweiligen Bohrung (32) des zweiten Anschlussteils (23) unter Vorspannung montiert ist.

10. Spritzenpaaranordnung (3; 3A, 3B, 3C, 3D) nach einem der Ansprüche, **dadurch gekennzeichnet, dass** der erste Anschlussteil (22) eine erste ebene Fläche (22A) aufweist, dass der zweite Anschlussteil (23) eine zweite ebene Fläche (23A) aufweist, wobei die erste und die zweite ebene Fläche (22A, 23A) einander zugewandt sind und zueinander parallel sind, wenn der erste und der zweite Anschlussteil (22, 23) miteinander verbunden sind, und dass das Verbindungselement (25) ausgelegt ist, um den ersten und den zweiten Anschlussteil (22, 23) unter Vorspannung miteinander zu verbinden, wobei die Vorspannung wenigstens im Wesentlichen quer zur ersten und zweiten ebenen Fläche (22A, 23A) gerichtet ist.

11. Spritzenpaaranordnung (3; 3A, 3B, 3C, 3D) nach einem der vorhergehenden Ansprüche, **dadurch gekennzeichnet, dass** das Halteelement (29) den Zylinderhalterahmen lösbar am zweiten Anschlussteil (23) sichert.

12. Spritzenpaaranordnung (3; 3A, 3B, 3C, 3D) nach einem der vorhergehenden Ansprüche, wobei die Spritzenpaaranordnung (3; 3A, 3B, 3C, 3D) anstelle von zwei Spritzen (12, 13) eine, drei oder mehr Spritzen umfasst.

## Revendications

1. Ensemble de seringues en paire (3 ; 3A, 3B, 3C, 3D) pour fluides biologiques comprenant deux seringues (12, 13), chaque seringue (12, 13) comprenant un corps cylindrique (14, 15), une buse (16, 17) et un piston plongeur (18, 19), un bâti de maintien de corps cylindrique (20) pour maintenir les corps cylindriques (14, 15), un support de piston plongeur (21) pour maintenir les pistons plongeurs (18, 19), un raccord (22, 23) avec un tubage (24), lequel raccord (22, 23) est adapté pour maintenir, par friction, les buses (16, 17) des deux seringues (12, 13) et pour relier les deux buses (16, 17) au tubage (24), un élément de retenue (29) fixant le bâti de maintien de corps cylindre au raccord (22, 23), une membrane (27) entourant les deux seringues (12, 13) et ayant une extrémité (26) scellée sur le raccord (22, 23) avec le tubage (24) et une autre extrémité fermée (28) opposée à la première extrémité (26) déployée autour des pistons plongeurs de seringue (18, 19) et adaptée pour permettre à un opérateur de saisir et de détendre les extrémités de piston plongeur pendant le remplissage des seringues (12, 13) sans les exposer aux conditions environnantes, ladite membrane étant formée à partir de parties (27, 27') qui sont scellées de manière séparable ensemble, **caractérisé en ce que** le raccord (22, 23) est formé par une première partie de raccord (22) et une seconde partie de raccord (23) qui sont raccordées de manière amovible entre elles au moyen d'un élément de raccordement (25), le tubage (24) et la première extrémité (26) de la membrane (27) étant fixés sur la première partie de raccord (22), la seconde partie de raccord (23) étant adaptée pour maintenir, par friction, les buses (16, 17) des deux seringues (12, 13), et **en ce que** l'ensemble (3 ; 3A, 3B, 3C, 3D) comprend en outre une membrane interne (27') positionnée à l'intérieur de la membrane (27), ladite membrane interne (27') entourant les deux seringues (12, 13) et ayant une extrémité (26') scellée sur la seconde partie de raccord (23) et une autre extrémité fermée (28') opposée à la première extrémité (26') déployée autour des pistons plongeurs de seringue (18, 19) et adaptée pour permettre à un opérateur de saisir et de détendre les extrémités de piston plongeur pendant le remplissage des seringues (12, 13) sans les exposer aux conditions environnantes, ladite membrane interne (27) étant formée à partir de parties qui sont scellées de manière séparable ensemble.

2. Ensemble de seringues en paire (3 ; 3A, 3B, 3C, 3D) selon la revendication 1, **caractérisé en ce que** l'élément de raccordement (25) est adapté pour raccorder la première et la seconde partie de raccord (22, 23) sous pré-tension.

3. Ensemble de seringues en paire (3 ; 3A, 3B, 3C, 3D) selon la revendication 1 ou 2, **caractérisé en ce que** ledit élément de raccordement (25) est adapté pour raccorder la première et la seconde partie de raccord (22, 23) sous une pré-tension exerçant une force dans une seule direction.

4. Ensemble de seringues en paire (3 ; 3A, 3B, 3C, 3D) selon la revendication 3, **caractérisé en ce que** l'élément de raccordement (25) comprend une première partie d'élément de raccordement (25A) prévue sur la première partie de raccord (22) et une seconde partie d'élément de raccordement (25B) prévue sur la seconde partie de raccordement (23), l'une (25A) des première et seconde parties d'élément de raccordement (25A, 25B) étant formée par un verrou pivotant (25A) et l'autre (25B) des première et seconde parties d'élément de raccordement (25A, 25B) étant formée par un cliquet (25B), ledit verrou pivotant (25A) et ledit cliquet (25B) étant adaptés pour raccorder la première et la seconde partie de raccord (22, 23) sous pré-tension, et ledit verrou pivotant (25A) comprenant un levier de poussée (30).

5. Ensemble de seringues en paire (3 ; 3A, 3B, 3C, 3D) selon l'une quelconque des revendications précédentes, **caractérisé en ce que** pour chaque seringue (12, 13), la première partie de raccord (22) comprend un tuyau en saillie (31) au niveau d'un côté de la première partie de raccord (22) opposée au tubage (24) et la seconde partie de raccord (23) comprend un trou (32) recevant le tuyau en saillie (31), ledit trou (32) étant en communication de fluide avec la buse (16, 17) de la seringue (12, 13) respective.

6. Ensemble de seringues en paire (3 ; 3A, 3B, 3C, 3D) selon l'une quelconque des revendications précédentes, **caractérisé en ce que** la seconde partie de raccord (23) est prévue avec une valve (33, 34) pour chaque buse (16, 17), ladite valve (33, 34) étant adaptée pour permettre la communication de fluide entre le tubage (24) et la buse (16, 17) respective, lorsque la première partie de raccord (22) et la seconde partie de raccord (23) sont raccordées entre elles et pour fermer la buse (16, 17) lorsque la première partie de raccord (22) et la seconde partie de raccord (23) sont déconnectées l'une de l'autre.

7. Ensemble de seringues en paire (3 ; 3A, 3B, 3C, 3D) selon les revendications 5 ou 6, **caractérisé en ce que** dans chaque trou (32) de la seconde partie de raccord (23), on positionne une valve (33, 34), et **en ce que** l'élément de raccordement (25), les première et seconde parties de raccord (22, 23) et la valve (33, 34) sont adaptés pour fermer automatiquement le trou (32) lorsque la première partie de raccord (22) et la seconde partie de raccord (23) sont déconnectées l'une de l'autre.

8. Ensemble de seringues en paire (3 ; 3A, 3B, 3C, 3D) selon la revendication 6 ou 7, **caractérisé en ce que** la valve (33, 34) est un manchon de valve flexible (33) recevant et entourant le tuyau en saillie (31).

9. Ensemble de seringues en paire (3 ; 3A, 3B, 3C, 3D) selon la revendication 6 ou 7, **caractérisé en ce que** chaque valve (33, 34) est une valve rotative (34) montée sous pré-tension dans le trou (32) respectif de la seconde partie de raccord (23).

10. Ensemble de seringues en paire (3 ; 3A, 3B, 3C, 3D) selon l'une quelconque des revendications, **caractérisé en ce que** la première partie de raccord (22) a une première surface plate (22A), **en ce que** la seconde partie de raccord (23) a une seconde surface plate (23A), les première et seconde surfaces plates (22A, 23A) se faisant face et étant parallèles entre elles lorsque les première et seconde parties de raccord (22, 23) sont raccordées entre elles, et **en ce que** l'élément de raccordement (25) est adapté pour raccorder la première et la seconde partie de raccord (22, 23) sous pré-tension, laquelle pré-tension est dirigée au moins sensiblement transversalement par rapport aux première et seconde surfaces plates (22A, 23A).

11. Ensemble de seringues en paire (3 ; 3A, 3B, 3C, 3D) selon l'une quelconque des revendications précédentes, **caractérisé en ce que** l'élément de retenue (29) fixe, de manière amovible, le bâti de maintien de corps cylindrique à la seconde partie de raccord (23).

12. Ensemble de seringues en paire (3 ; 3A, 3B, 3C, 3D) selon l'une quelconque des revendications précédentes, dans lequel, au lieu des deux seringues (12, 13), l'ensemble de seringues en paire (3 ; 3A, 3B, 3C, 3D) comprend une, trois seringues ou plus.
